# EUROPÄISCHE PATENTSCHRIFT

(11) **EP 4 021 375 B1**
(45) Veröffentlichungstag und Bekanntmachung des Hinweises auf die Patenterteilung: **21.05.2025**
(21) Anmeldenummer: 20764043.4
(22) Anmeldetag: 21.08.2020
(51) Int. Cl.: A61K 8/04, A61Q 17/04, A61K 8/73, A61K 8/49, A61K 8/41

(54) **ETHYLCELLULOSE ENTHALTENDES SONNENSCHUTZSPRAY**
SUNSCREEN SPRAY CONTAINING ETHYL CELLULOSE
SPRAY DE PROTECTION SOLAIRE CONTENANT DE L'ÉTHYLCELLULOSE

(30) Priorität: 28.08.2019 DE 102019212924
(43) Veröffentlichungstag der Anmeldung: 06.07.2022
(73) Patentinhaber: Beiersdorf AG, 22529 Hamburg (DE)
(72) Erfinder: NILSSON, Jan, 22177 Hamburg (DE); BLECKMANN, Andreas, 22926 Ahrensburg (DE); PEIRANO, Reto, 22761 Hamburg (DE)
(74) Vertreter: Beiersdorf AG
(86) Internationale Anmeldenummer: PCT/EP2020/073492
(87) Internationale Veröffentlichungsnummer: WO 2021/037717

(56) Entgegenhaltungen:
- EP-A2- 1 291 009
- WO-A1-2016/008121
- JP-A- 2019 108 303

## Beschreibung

Die vorliegende Erfindung betrifft die Verwendung von Ethylcellulose in kosmetischen, ethanolischen Sonnenschutzsprays zur Verhinderung der Lungengängigkeit der Spraytröpfchen, sowie kosmetische Zubereitungen in Form eines Sonnenschutzsprays enthaltend Ethanol und Ethylcellulose sowie Ethylhexyl Triazone und/oder Hexyl 2-[4-(diethylamino)-2-hydroxybenzoyl]benzoate (INCI: Diethylamino hydroxybenzoyl hexyl benzoate).

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Eine beliebte Anwendungsform kosmetischer Sonnenschutzmittel sind die Sonnenschutzsprays, bei denen die Zubereitung entweder mit einer Kolbenhub-Pumpe oder einem Treibgas aus dem Vorratsbehältnis unter Druck durch einen Sprühkopf gepresst und so in Form fein verteilter Tröpfchen auf die Haut aufgetragen werden. Bei dieser Anwendungsform kann üblicherweise auf ein weiteres Verreiben und Verteilen der Zubereitung auf der Haut verzichtet werden. Diese fein verteilten Tröpfchen werden üblicherweise als Aerosole bezeichnet.

Nachteilig am Stande der Technik ist nun der Umstand dass diese Aerosole bei der Applikation des Sonnenschutzmittels vom Anwender eingeatmet werden können.

Werden Aerosole vom Menschen eingeatmet, scheidet sich ein Teil der inhalierten Aerosolpartikel im Atemtrakt ab. Ungefähr 10 % aller inhalierten Aerosolteilchen bleiben im Atemtrakt. Da die Abscheidewahrscheinlichkeit eines Teilchens stark von seiner Größe abhängt, ist dies allerdings nur ein grober Richtwert. Teilchen, die mindestens bis in den Bronchialbereich vordringen können, heißen lungengängig. Dazu gehören alle Aerosolpartikel unterhalb eines Durchmessers von ungefähr 10 Mikrometer (PM10). Größere Teilchen scheiden sich schon in der Nase oder im Rachen ab oder lassen sich überhaupt nicht inhalieren. Am wenigsten scheiden sich Teilchen mit einem Durchmesser zwischen 0,5 Mikrometer und 1 Mikrometer ab. Das bedeutet gleichzeitig, dass sie besonders tief in die Lunge eindringen. Deutlich größere und kleinere Teilchen scheiden sich bereits in den oberen Bereichen stärker ab, dringen dadurch weniger tief ein und belasten die empfindlichen Alveolen weniger (Wikipedia).

Die Größe von Aerosol-Tröpfchen hängt bei Sprayformulierungen üblicherweise vom Sprühkopf, dem Sprühdruck und der Zusammensetzung der versprühten Zubereitung ab.

Es war nun die Aufgabe der vorliegenden Erfindung, ein Sonnenschutzspray zu entwickeln, bei dem rezepturseitig die Zubereitung dahingehend verbessert werden sollte, dass die Aerosol-Tröpfchen (unter Verwendung von Standard-Sprühköpfen und herkömmlichen Sprühdrücken von 2,0 bis 4,0 bar, bevorzugt 2,7 bar) nicht mehr lungengängig sind, d.h. dass die Tröpfchengröße der Aerosol-Tröpfchen mindestens 10 Mikrometer betragen sollte und allenfalls 0,2% der Tröpfchen eine kleinere Tröpfchengröße aufweisen durften.

Überraschend gelöst wird die Aufgabe durch die Verwendung von Ethylcellulose in kosmetischen, ethanolischen Sonnenschutzsprays zur Verhinderung der Lungengängigkeit der Spraytröpfchen, dadurch gekennzeichnet, dass das kosmetische, ethanolische Sonnenschutzspray 2,4,6-Tris-[anilino-(p-carbo-2'-ethyl-1'-hexyloxy)]-1,3,5-triazin (INCI: Ethylhexyl Triazone) und/oder Hexyl 2-[4-(diethylamino)-2-hydroxybenzoyl]benzoate (INCI: Diethylamino hydroxybenzoyl hexyl benzoate) enthält, wobei Aerosole als nicht mehr lungengängig gelten, deren Tröpfchengröße mindestens 10 Mikrometer beträgt und allenfalls 0,2% der Tröpfchen eine kleinere Tröpfchengröße aufweisen.

Daher wird die Aufgabe der vorliegenden Erfindung ferner gelöst durch eine kosmetische Zubereitung in Form eines Sonnenschutzspray enthaltend
a) Ethanol,
b) Ethylcellulose sowie
c) 2,4,6-Tris-[anilino-(p-carbo-2'-ethyl-1'-hexyloxy)]-1,3,5-triazin (INCI: Ethylhexyl Triazone) und/oder Hexyl 2-[4-(diethylamino)-2-hydroxybenzoyl]benzoate (INCI: Diethylamino hydroxybenzoyl hexyl benzoate), dadurch gekennzeichnet, dass das Sonnenschutzspray Ethylcellulose in einer Konzentration von 0,1 bis 2 Gewichts-%, bezogen auf das Gesamtgewicht der Zubereitung enthält.

Erfindungsgemäß wurden dabei die Zubereitungen mit und ohne Ethylcellulose unter Verwendung eines Standardsprühkopfes (Standardsprühköpfe P72, Helios 5027 oder 4061 der Firma Aptar) und einem Sprühdruck von 2,7 bar Treibgas 60% Butane, 20% Isobutane, 20% Propane miteinander verglichen.

Als nicht mehr lungengängig gelten im Rahmen dieser Offenbarung Aerosole, deren Tröpfchengröße unter den oben angegebenen Messbedingungen mindestens 10 Mikrometer beträgt und allenfalls 0,2% der Tröpfchen eine kleinere Tröpfchengröße aufweisen.

Im Rahmen der vorliegenden Offenbarung beziehen sich die Formulierungen "erfindungsgemäß", "erfindungsgemäße Zubereitung" etc. immer auf die erfindungsgemäßen Zubereitungen und Verwendungen, d.h. auch auf Zubereitungen, in denen die erfindungsgemäßen Verwendungen verwirklicht werden.

Neben dem Einsatz in Aerosol-Spraydosen ist es ebenso erfindungsgemäß, die erfindungsgemäße Verwendung bzw. Zubereitung in sogenannten Bag on Valve Systemen einzusetzen Hier liegt der Druck bei 7-10 Bar im Vorratsgefäß.

Zwar kennt der Stand der Technik die WO 2016/008121 A1, die JP 2019 108303A sowie die EP 1 291 009 A2, doch konnten diese Schriften nicht den Weg zur vorliegenden Erfindung weisen.

Es ist für die erfindungsgemäße Verwendung erfindungsgemäß bevorzugt, wenn das Sonnenschutzspray Ethylcellulose in einer Konzentration von 0,1 bis 2,0 Gewichts-%, bezogen auf das Gesamtgewicht der Zubereitung enthält. Bei höheren Einsatzkonzentrationen hingegen verschlechtert sich das Sprühbild. Dies liegt möglicherweise an den verdickenden Eigenschaften der Ethylcellulose, die bei höheren Einsatzkonzentrationen zum Tragen kommen.

Es ist erfindungsgemäß von Vorteil, wenn das Sonnenschutzspray Ethanol in einer Konzentration von 20 bis 80 Gewichts-%, bevorzugt von 30 bis 70 Gewichts-% und besonders bevorzugt von 40 bis 40 Gewichts-%, jeweils bezogen auf das Gesamtgewicht der Zubereitung, enthält.

Im Zusammenhang mit den UV-Filtern Ethylhexyl Triazone und Diethylamino hydroxybenzoyl hexyl benzoate können erfindungsgemäß drei Fälle unterschieden werden: Zubereitungen, die Ethylhexyl Triazone enthalten, Zubereitungen, die Diethylamino hydroxybenzoyl hexyl benzoate enthalten sowie Zubereitungen, welche Ethylhexyl Triazone und Diethylamino hydroxybenzoyl hexyl benzoate enthalten. Für alle diese Ausführungsformen gelten die folgenden Einsatzkonzentrationen als erfindungsgemäß vorteilhaft:
Erfindungsgemäß vorteilhafte Ausführungsformen der vorliegenden Erfindung sind dadurch gekennzeichnet, dass die Zubereitung 2,4,6-Tris-[anilino-(p-carbo-2'-ethyl-1'-hexyloxy)]-1,3,5-triazin (INCI: Ethylhexyl Triazone) in einer Konzentration von 0,5 bis 2,5 Gewichts-%, bezogen auf das Gesamtgewicht der Zubereitung, enthält.

Vorteilhafte Ausführungsformen im Sinne der vorliegenden Erfindung sind dadurch gekennzeichnet, dass die Zubereitung Hexyl 2-[4-(diethylamino)-2-hydroxybenzoyl]benzoate (INCI: Diethylamino hydroxybenzoyl hexyl benzoate) in einer Konzentration von 0,5 bis 2,5 Gewichts-%, bezogen auf das Gesamtgewicht der Zubereitung, enthält.

Vorteilhafte Ausführungsformen der vorliegenden Erfindung sind dadurch gekennzeichnet, dass die Ethylcellulose eine Glasübergangstemperatur von 129-133 °C aufweist. Sie ist ferner als erfindungsgemäß vorteilhaft zu kennzeichnen, wenn sie einen Brechungsindex von 1,47 aufweist.

Außerdem ist es vorteilhaft im Sinne der vorliegenden Erfindung, wenn die Ethylcellulose einen Ethoxyl-Gehalt von 48-49,5 Gewichts-% aufweist.

Vorteilhafte Ausführungsformen der vorliegenden Erfindung sind dadurch gekennzeichnet, dass die Ethylcellulose ein mittleres Molekulargewicht (Gewichtsmittel) M_{w} von 215000, gemessen mittels Größenausschlusschromatographie, aufweist.

Als erfindungsgemäß vorteilhafte Ethylcellulosen können beispielsweise eingesetzt werden, die Ethocel Standard^{®} Produkte der Firma Dow Chemical Company oder die Ethylcellulose EC N200 PC^{®} der Firma Aqualon.

Die erfindungsgemäße Zubereitung enthält üblicherweise weitere UV-Filter.

Dabei ist es erfindungsgemäß vorteilhaft, wenn die Zubereitung 4-(tert.-Butyl)-4'-methoxydibenzoylmethan enthält.

Die erfindungsgemäß vorteilhafte Einsatzkonzentration für 4-(tert.-Butyl)-4'-methoxydibenzoylmethan beträgt von 1 bis 5 Gewichts-%, bezogen auf das Gesamtgewicht der Zubereitung.

Außerdem ist es erfindungsgemäß von Vorteil, wenn die Zubereitung einen oder mehrere UV-B-Filter gewählt aus der Gruppe der Verbindungen 2-Ethylhexyl 2-hydroxybenzoat (INCI: Ethylhexyl Salicylate) und 3,3,5-Trimethylcyclohexyl 2-hydroxybenzoat (INCI: Homosalate) enthält.

Die erfindungsgemäß vorteilhafte Einsatzkonzentration für 2-Ethylhexyl 2-hydroxybenzoat (INCI: Ethylhexyl Salicylate) beträgt von 1 bis 6 Gewichts-%, bezogen auf das Gesamtgewicht der Zubereitung.

Die erfindungsgemäß vorteilhafte Einsatzkonzentration für 3,3,5-Trimethylcyclohexyl 2-hydroxybenzoat (INCI: Homosalate) beträgt von 1 bis 10 Gewichts-%, bezogen auf das Gesamtgewicht der Zubereitung.

Ferner sind die erfindungsgemäß vorteilhaften Ausführungsformen der vorliegenden Erfindung dadurch gekennzeichnet, dass die Zubereitung 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) enthält.

Die erfindungsgemäß vorteilhafte Einsatzkonzentration für 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) beträgt von 0,5 bis 5 Gewichts-%, bezogen auf das Gesamtgewicht der Zubereitung.

Darüber hinaus kann es erfindungsgemäß von Vorteil sein, wenn die Zubereitung Ethylhexyl-2-cyano-3,3-diphenylacrylat (INCI: Octocrylen) enthält.

In einem solchen Falle beträgt die erfindungsgemäß vorteilhafte Einsatzkonzentration für Ethylhexyl-2-cyano-3,3-diphenylacrylat (INCI: Octocrylen) beträgt von 2,5 bis 10 Gewichts-%, bezogen auf das Gesamtgewicht der Zubereitung.

In jedem Falle ist es erfindungsgemäß vorteilhaft, wenn die Zubereitung kein 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester, keine Parabene, Methylisothiazolinon, Chlormethylisothiazolinon und DMDM-Hydantoin enthält.

Die erfindungsgemäß vorteilhaften Ausführungsformen sind hingegen dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist der Einsatz von Dibutyladipat, Propylheptyl Caprylate und C12-15 Alkyl Benzoat erfindungsgemäß bevorzugt.

Eine besonders vorteilhafte Ausführungsform der vorliegenden Erfindung ist dadurch gekennzeichnet, dass die Zubereitung Acrylat/Octylacrylamid Copolymer (INCI: Acrylates/Octylacrylamide Copolymer) und höchstens 2,5 Gewichts-%, bezogen auf das Gesamtgewicht der Zubereitung, an Glycerin enthält. Die Einsatzkonzentration für Acrylat/Octylacrylamid Copolymer (INCI: Acrylates/Octylacrylamide Copolymer) beträgt dabei vorteilhafterweise von 0,2 bis 4 Gewichts-%, bezogen auf das Gesamtgewicht der Zubereitung.

Beträgt die Einsatzkonzentration von Glycerin hingegen mehr als 2,5 Gewichts-%, bezogen auf das Gesamtgewicht der Zubereitung, so wird erfindungsgemäß bevorzugt auf den Einsatz von Acrylat/Octylacrylamid Copolymer (INCI: Acrylates/Octylacrylamide Copolymer) verzichtet.

Es ist erfindungsgemäß vorteilhaft, wenn die Zubereitung wasserfrei ist. Dabei gelten Zubereitungen erfindungsgemäß als wasserfrei, wenn der Wassergehalt der Zubereitung weniger als 3 Gewichts-%, bezogen auf das Gesamtgewicht der Zubereitung, beträgt.

Erfindungsgemäß vorteilhafte Ausführungsformen der vorliegenden Erfindung sind ferner dadurch gekennzeichnet, dass die Zubereitung frei ist von Titandioxid, Zinkoxid und Polysilicon-15.

Es ist erfindungsgemäß vorteilhaft, wenn die Zubereitung mit einem Aerosol-Druckgas-Behälter aufbewahrt und mit Hilfe eines Treibgases versprüht wird. In einem solchen Falle ist es erfindungsgemäß bevorzugt, wenn als Treibgas Propan, n-Butan, Isobutan oder deren Mischungen eingesetzt werden.

### Vergleichsversuch

Es wurden die folgenden Rezepturen hergestellt und die Tröpfchengrößenverteilung mit Hilfe der folgenden Methode bestimmt.

### Messmethode

Characterization of Sunscreen Sprays beim Fraunhofer Institute for Toxicology and Experimental Medicine ITEM

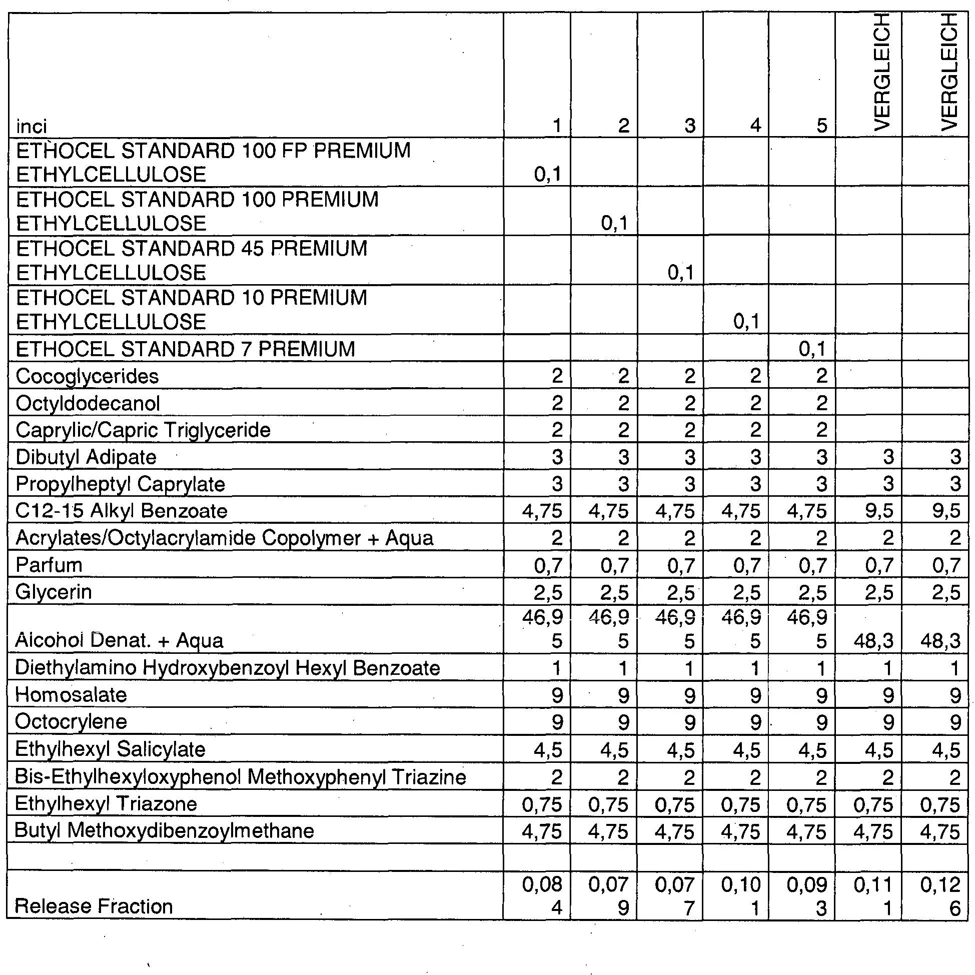

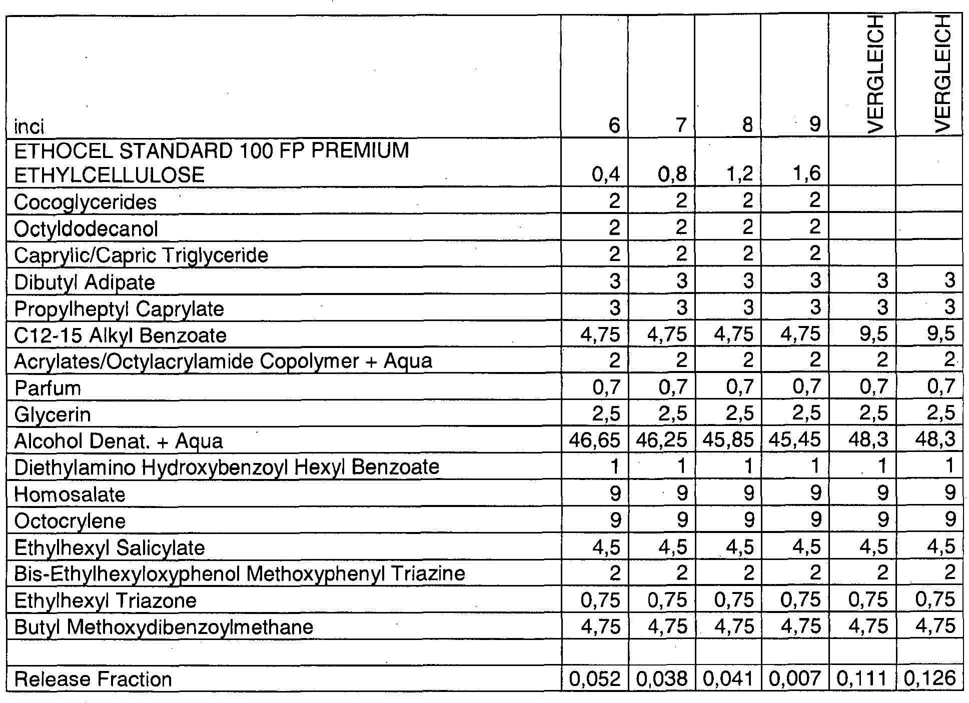

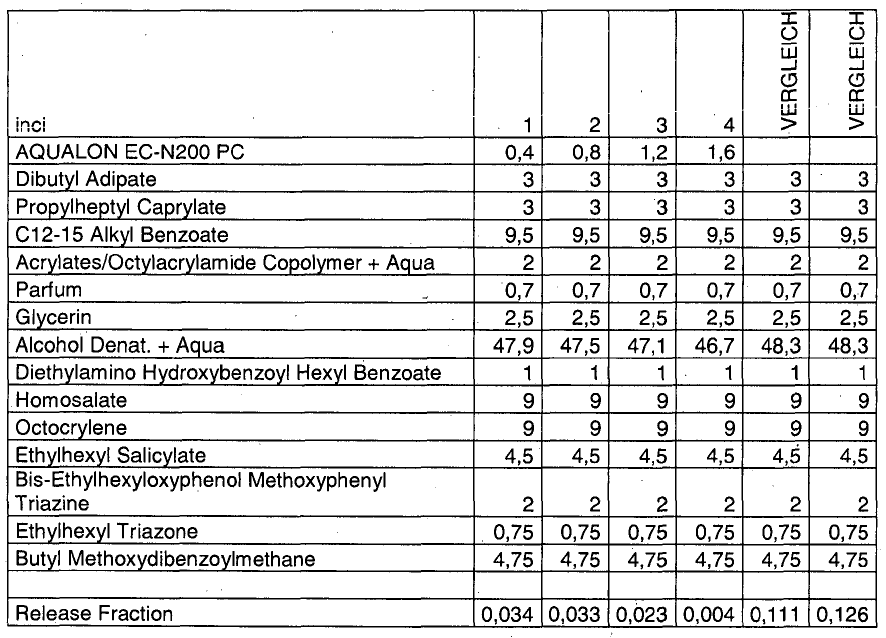

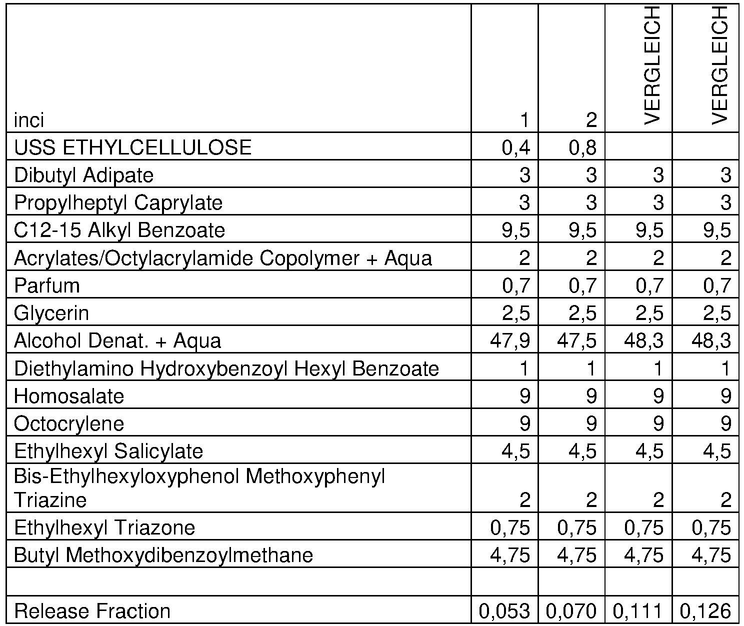

Die Release Fraction ist definiert als die Masse m des gealterten Sprays/Aerosols im Bereich von 0,6-10µm normalisiert auf die Gesamtmasse des freigesetzten Sprühprodukts (*RF = m*/*M*).

Die Release Fraction charakterisiert den Auftragungsprozess durch Schätzung der Inhalationsexposition für ein definiertes Anwendungsszenario. Sie wird bestimmt, indem das Produkt über einen kurzen Zeitraum in ein genau definiertes Kontrollvolumen gesprüht und zeitaufgelöste Messungen der Tröpfchenkonzentration durchgeführt werden.

### Beispiele

Die nachfolgenden Beispiele, ausser 2,3,6 und 12, denen die Filter Ethylhexyl Triazone (INCI) und/oder Diethylamino hydroxybenzoyl hexyl benzoate (INCI) fehlen, sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

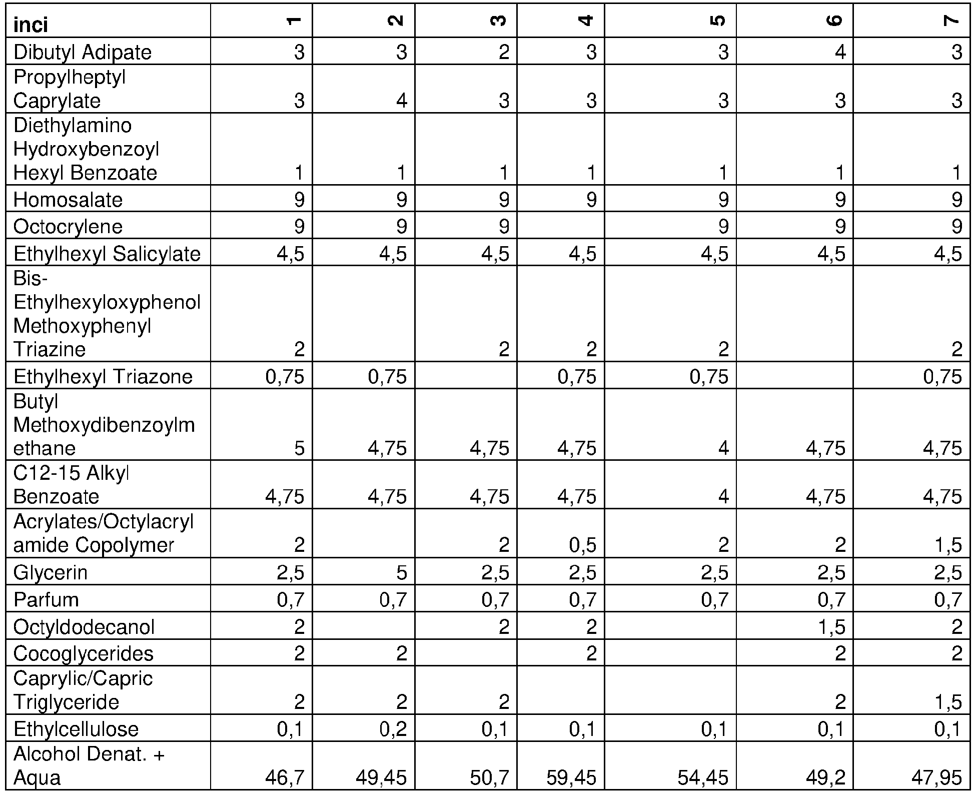

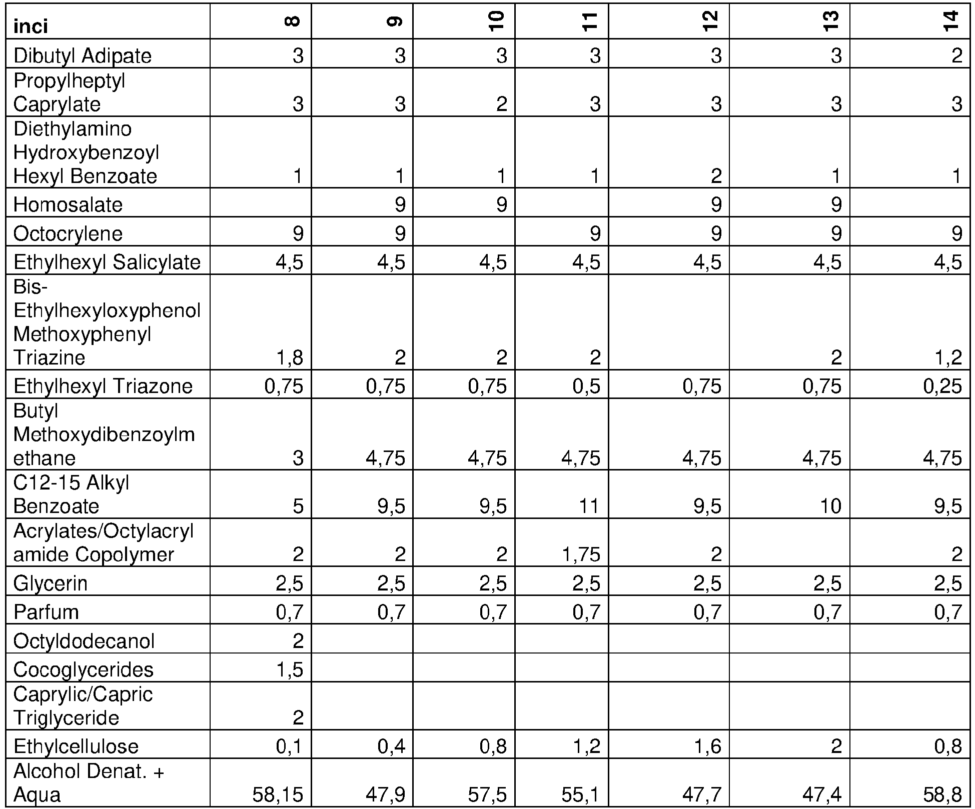

## Patentansprüche

1. Verwendung von Ethylcellulose in kosmetischen, ethanolischen Sonnenschutzsprays zur Verhinderung der Lungengängigkeit der Spraytröpfchen, **dadurch gekennzeichnet, dass** das kosmetische, ethanolische Sonnenschutzspray 2,4,6-Tris-[anilino-(p-carbo-2'-ethyl-1'-hexyloxy)]-1,3,5-triazin (INCI: Ethylhexyl Triazone) und/oder Hexyl 2-[4-(diethylamino)-2-hydroxybenzoyl]benzoate (INCI: Diethylamino hydroxybenzoyl hexyl benzoate) enthält, wobei Aerosole als nicht mehr lungengängig gelten, deren Tröpfchengröße mindestens 10 Mikrometer beträgt und allenfalls 0,2% der Tröpfchen eine kleinere Tröpfchengröße aufweisen.

2. Kosmetische Zubereitung in Form eines Sonnenschutzspray enthaltend
a) Ethanol,
b) Ethylcellulose sowie
c) 2,4,6-Tris-[anilino-(p-carbo-2'-ethyl-1'-hexyloxy)]-1,3,5-triazin (INCI: Ethylhexyl Triazone) und/oder Hexyl 2-[4-(diethylamino)-2-hydroxybenzoyl]benzoate (INCI: Diethylamino hydroxybenzoyl hexyl benzoate), **dadurch gekennzeichnet, dass** das Sonnenschutzspray Ethylcellulose in einer Konzentration von 0,1 bis 2 Gewichts-%, bezogen auf das Gesamtgewicht der Zubereitung enthält.

3. Verwendung nach Anspruch 1, **dadurch gekennzeichnet, dass** das Sonnenschutzspray Ethylcellulose in einer Konzentration von 0,1 bis 2 Gewichts-%, bezogen auf das Gesamtgewicht der Zubereitung enthält.

4. Verwendung oder Zubereitung nach einem der vorhergehenden Ansprüche, **dadurch gekennzeichnet, dass** das Sonnenschutzspray Ethanol in einer Konzentration von 20 bis 80 Gewichts-%, bezogen auf das Gesamtgewicht der Zubereitung, enthält.

5. Verwendung oder Zubereitung nach einem der vorhergehenden Ansprüche, **dadurch gekennzeichnet, dass** die Zubereitung 2,4,6-Tris-[anilino-(p-carbo-2'-ethyl-1'-hexyl-oxy)]-1,3,5-triazin (INCI: Ethylhexyl Triazone) in einer Konzentration von 0,5 bis 2,5 Gewichts-%, bezogen auf das Gesamtgewicht der Zubereitung, enthält.

6. Verwendung oder Zubereitung nach einem der vorhergehenden Ansprüche, **dadurch gekennzeichnet, dass** die Zubereitung Hexyl 2-[4-(diethylamino)-2-hydroxybenzoyl]benzoate (INCI: Diethylamino hydroxybenzoyl hexyl benzoate) in einer Konzentration von 0,5 bis 2,5 Gewichts-%, bezogen auf das Gesamtgewicht der Zubereitung, enthält.

7. Verwendung oder Zubereitung nach einem der vorhergehenden Ansprüche, **dadurch gekennzeichnet, dass** die Ethylcellulose eine Glasübergangstemperatur von 129-133 °C aufweist.

8. Verwendung oder Zubereitung nach einem der vorhergehenden Ansprüche, **dadurch gekennzeichnet, dass** die Ethylcellulose einen Brechungsindex von 1,47 aufweist.

9. Verwendung oder Zubereitung nach einem der vorhergehenden Ansprüche, **dadurch gekennzeichnet, dass** die Ethylcellulose einen Ethoxyl-Gehalt von 48.0-49,5 Gewichts-% aufweist.

10. Verwendung oder Zubereitung nach einem der vorhergehenden Ansprüche, **dadurch gekennzeichnet, dass** die Zubereitung 4-(tert.-Butyl)-4'-methoxydibenzoylmethan enthält.

11. Verwendung oder Zubereitung nach einem der vorhergehenden Ansprüche, **dadurch gekennzeichnet, dass** die Zubereitung einen oder mehrere UV-B-Filter gewählt aus der Gruppe der Verbindungen 2-Ethylhexyl 2-hydroxybenzoat (INCI: Ethylhexyl Salicylate) und 3,3,5-Trimethylcyclohexyl 2-hydroxybenzoat (INCI: Homosalate) enthält.

12. Verwendung oder Zubereitung nach einem der vorhergehenden Ansprüche, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) enthält.

13. Verwendung oder Zubereitung nach einem der vorhergehenden Ansprüche, **dadurch gekennzeichnet, dass** die Zubereitung Ethylhexyl-2-cyano-3,3-diphenylacrylat (INCI: Octocrylen) enthält.

14. Verwendung oder Zubereitung nach einem der vorhergehenden Ansprüche, **dadurch gekennzeichnet, dass** die Zubereitung kein 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester, keine Parabene, Methylisothiazolinon, Chlormethylisothiazolinon und DMDM-Hydantoin enthält.

15. Verwendung oder Zubereitung nach einem der vorhergehenden Ansprüche, **dadurch gekennzeichnet, dass** die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

16. Verwendung oder Zubereitung nach einem der vorhergehenden Ansprüche, **dadurch gekennzeichnet, dass** die Zubereitung Acrylat/Octylacrylamid Copolymer (INCI: Acrylates/Octylacrylamide Copolymer) und höchstens 2,5 Gewichts-%, bezogen auf das Gesamtgewicht der Zubereitung, an Glycerin enthält.

17. Verwendung oder Zubereitung nach einem der vorhergehenden Ansprüche, **dadurch gekennzeichnet, dass** die Zubereitung wasserfrei ist.

18. Verwendung oder Zubereitung nach einem der vorhergehenden Ansprüche, **dadurch gekennzeichnet, dass** die Zubereitung frei ist von Titandioxid, Zinkoxid und Polysilicon-15.

19. Verwendung oder Zubereitung nach einem der vorhergehenden Ansprüche, **dadurch gekennzeichnet, dass** die Zubereitung mit einem Aerosol-Druckgas-Behälter aufbewahrt und mit Hilfe eines Treibgases versprüht wird.

20. Verwendung oder Zubereitung nach Anspruch 20, **dadurch gekennzeichnet, dass** als Treibgas Propan, n-Butan, Isobutan oder deren Mischungen eingesetzt werden.

## Claims

1. Use of ethylcellulose in cosmetic, ethanolic sunscreen sprays to prevent the respirability of the spray droplets, **characterized in that** the cosmetic, ethanolic sunscreen spray comprises 2,4,6-tris[anilino-(p-carbo-2'-ethyl-1'-hexyloxy)]-1,3,5-triazine (INCI: Ethylhexyl Triazone) and/or hexyl 2-[4-(diethylamino)-2-hydroxybenzoyl]benzoate (INCI: Diethylamino Hydroxybenzoyl Hexyl Benzoate), where aerosols are no longer considered to be respirable when they have a droplet size of at least 10 micrometres and at most 0.2% of the droplets have a smaller droplet size.

2. Cosmetic preparation in the form of a sunscreen spray comprising
a) ethanol,
b) ethylcellulose, and
c) 2,4,6-tris[anilino-(p-carbo-2'-ethyl-1'-hexyloxy)]-1,3,5-triazine (INCI: Ethylhexyl Triazone) and/or hexyl 2-[4-(diethylamino)-2-hydroxybenzoyl]benzoate (INCI: Diethylamino Hydroxybenzoyl Hexyl Benzoate), **characterized in that** the sunscreen spray comprises ethylcellulose in a concentration of 0.1% to 2% by weight, based on the total weight of the preparation.

3. Use according to Claim 1, **characterized in that** the sunscreen spray comprises ethylcellulose in a concentration of 0.1% to 2% by weight, based on the total weight of the preparation.

4. Use or preparation according to any of the preceding claims, **characterized in that** the sunscreen spray comprises ethanol in a concentration of 20% to 80% by weight, based on the total weight of the preparation.

5. Use or preparation according to any of the preceding claims, **characterized in that** the preparation comprises 2,4,6-tris[anilino-(p-carbo-2'-ethyl-1'-hexyloxy)]-1,3,5-triazine (INCI: Ethylhexyl Triazone) in a concentration of 0.5% to 2.5% by weight, based on the total weight of the preparation.

6. Use or preparation according to any of the preceding claims, **characterized in that** the preparation comprises hexyl 2-[4-(diethylamino)-2-hydroxybenzoyl]benzoate (INCI: Diethylamino Hydroxybenzoyl Hexyl Benzoate) in a concentration of 0.5% to 2.5% by weight, based on the total weight of the preparation.

7. Use or preparation according to any of the preceding claims, **characterized in that** the ethylcellulose has a glass transition temperature of 129-133°C.

8. Use or preparation according to any of the preceding claims, **characterized in that** the ethylcellulose has a refractive index of 1.47.

9. Use or preparation according to any of the preceding claims, **characterized in that** the ethylcellulose has an ethoxyl content of 48.0-49.5% by weight.

10. Use or preparation according to any of the preceding claims, **characterized in that** the preparation comprises 4-(tert-butyl)-4'-methoxydibenzoylmethane.

11. Use or preparation according to any of the preceding claims, **characterized in that** the preparation comprises one or more UV-B filters selected from the following group of compounds: 2-ethylhexyl 2-hydroxybenzoate (INCI: Ethylhexyl Salicylate) and 3,3,5-trimethylcyclohexyl 2-hydroxybenzoate (INCI: Homosalate).

12. Use or preparation according to any of the preceding claims, **characterized in that** the preparation comprises 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

13. Use or preparation according to any of the preceding claims, **characterized in that** the preparation comprises ethylhexyl 2-cyano-3,3-diphenylacrylate (INCI: Octocrylene).

14. Use or preparation according to any of the preceding claims, **characterized in that** the preparation does not comprise any 3-(4-methylbenzylidene)camphor, 2-hydroxy-4-methoxybenzophenone (INCI: Oxybenzone), 2-ethylhexyl 4-methoxycinnamate, isoamyl 4-methoxycinnamate, any parabens, methylisothiazolinone, chloromethylisothiazolinone and DMDM hydantoin.

15. Use or preparation according to any of the preceding claims, **characterized in that** the preparation comprises one or more oils selected from the following group of compounds: Butylene Glycol Dicaprylate/Dicaprate, Phenethyl Benzoate, C12-15 Alkyl Benzoate, dibutyl adipate; diisopropyl sebacate, dicaprylyl carbonate, Di-C12-13 Alkyl Tartrate, Butyloctyl Salicylate, Diethylhexyl Syringylidene Malonate, Hydrogenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate.

16. Use or preparation according to any of the preceding claims, **characterized in that** the preparation comprises acrylate/octylacrylamide copolymer (INCI: Acrylates/Octylacrylamide Copolymer) and at most 2.5% by weight, based on the total weight of the preparation, of glycerin.

17. Use or preparation according to any of the preceding claims, **characterized in that** the preparation is anhydrous.

18. Use or preparation according to any of the preceding claims, **characterized in that** the preparation is free of titanium dioxide, zinc oxide and Polysilicone-15.

19. Use or preparation according to any of the preceding claims, **characterized in that** the preparation is stored with an aerosol-pressurized gas container and sprayed using a propellant.

20. Use or preparation according to Claim 20, **characterized in that** the propellant used is propane, n-butane, isobutane or mixtures thereof.

## Revendications

1. Utilisation d'éthylcellulose dans des sprays de protection solaire cosmétiques, éthanoliques pour empêcher la pénétration dans les poumons des gouttelettes de spray, **caractérisées en ce que** le spray de protection solaire cosmétique, éthanolique contient de la 2,4,6-tris-[anilino-(p-carbo-2'-éthyl-1'-hexyloxy)]-1,3,5-triazine (INCI : Ethylhexyl Triazone) et/ou du 2-[4-(diéthylamino)-2-hydroxybenzoyl]benzoate d'hexyle (INCI : Diethylamino hydroxybenzoyl hexyl benzoate), les aérosols étant considérés comme ne pénétrant plus dans les poumons lorsque la grosseur des gouttelettes est d'au moins 10 micromètres et que, tout au plus, 0,2% des gouttelettes présentent une grosseur inférieure.

2. Préparation cosmétique sous forme d'un spray de protection solaire contenant
a) de l'éthanol,
b) de l'éthylcellulose ainsi que
c) de la 2,4,6-tris-[anilino-(p-carbo-2'-éthyl-1'-hexyloxy)]-1,3,5-triazine (INCI : Ethylhexyl Triazone) et/ou du 2-[4-(diéthylamino)-2-hydroxybenzoyl]benzoate d'hexyle (INCI : Diethylamino hydroxybenzoyl hexyl benzoate), **caractérisée en ce que** le spray de protection solaire contient de l'éthylcellulose en une concentration de 0,1 à 2% en poids, par rapport au poids total de la préparation.

3. Utilisation selon la revendication 1, **caractérisée en ce que** le spray de protection solaire contient de l'éthylcellulose en une concentration de 0,1 à 2% en poids, par rapport au poids total de la préparation.

4. Utilisation ou préparation selon l'une des revendications précédentes, **caractérisée en ce que** le spray de protection solaire contient de l'éthanol en une concentration de 20 à 80% en poids par rapport au poids total de la préparation.

5. Utilisation ou préparation selon l'une des revendications précédentes, **caractérisée en ce que** la préparation contient de la 2,4,6-tris-[anilino-(p-carbo-2'-éthyl-1'-hexyl-oxy)]-1,3,5-triazine (INCI : Ethylhexyl Triazone) en une concentration de 0,5 à 2,5% en poids, par rapport au poids total de la préparation.

6. Utilisation ou préparation selon l'une des revendications précédentes, **caractérisée en ce que** la préparation contient du 2-[4-(diéthylamino)-2-hydroxybenzoyl]benzoate d'hexyle (INCI : Diethylamino hydroxybenzoyl hexyl benzoate) en une concentration de 0,5 à 2,5% en poids, par rapport au poids total de la préparation.

7. Utilisation ou préparation selon l'une des revendications précédentes, **caractérisée en ce que** l'éthylcellulose présente une température de transition vitreuse de 129-133°C.

8. Utilisation ou préparation selon l'une des revendications précédentes, **caractérisée en ce que** l'éthylcellulose présente un indice de réfraction de 1,47.

9. Utilisation ou préparation selon l'une des revendications précédentes, **caractérisée en ce que** l'éthylcellulose présente une teneur en éthoxyle de 48,0-49,5% en poids.

10. Utilisation ou préparation selon l'une des revendications précédentes, **caractérisée en ce que** la préparation contient du 4-(tert-butyl)-4'-méthoxydibenzoylméthane.

11. Utilisation ou préparation selon l'une des revendications précédentes, **caractérisée en ce que** la préparation contient un ou plusieurs filtres UV-B choisis dans le groupe des composés 2-hydroxybenzoate de 2-éthylhexyle (INCI : Ethylhexyl Salicylate) et 2-hydroxybenzoate de 3,3,5-triméthylcyclohexyle (INCI : Homosalate).

12. Utilisation ou préparation selon l'une des revendications précédentes, **caractérisée en ce que** la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol methoxyphenyl Triazine).

13. Utilisation ou préparation selon l'une des revendications précédentes, **caractérisée en ce que** la préparation contient du 2-cyano-3,3-diphénylacrylate d'éthylhexyle (INCI : Octocrylen).

14. Utilisation ou préparation selon l'une des revendications précédentes, **caractérisée en ce que** la préparation ne contient pas de 3-(4-méthylbenzylidène)-camphre, de 2-hydroxy-4-méthoxybenzophénone (INCI : Oxybenzon), d'ester (2-éthylhexylique) de l'acide 4-méthoxycinnamique, d'ester isoamylique de l'acide 4-méthoxycinnamique, pas de parabènes, de méthylisothiazolinone, de chlorométhylisothiazolinone et de DMDM-hydantoïne.

15. Utilisation ou préparation selon l'une des revendications précédentes, **caractérisée en ce que** la préparation contient une ou plusieurs huiles choisies dans le groupe des composés dicaprylate/dicaprate de butylèneglycol, benzoate de phénéthyle, benzoate de C12-15-alkyle, adipate de dibutyle ; sébacate de diisopropyle, carbonate de dicaprylyle, tartrate de di-C12-13-alkyle, salicylate de butyloctyle, syringylidènemalonate de diéthylhexyle, dimérate d'huile de ricin hydrogénée, triheptanoïne, lactate de C12-13-alkyle, benzoate de C16-17-alkyle, caprylate de propylheptyle, triglycéride caprylique/caprique, 2,6-naphtalate de diéthylhexyle, octyldodécanol, triglycéride caprylique/caprique, cocoate d'éthylhexyle.

16. Utilisation ou préparation selon l'une des revendications précédentes, **caractérisée en ce que** la préparation contient un copolymère d'acrylate/octylacrylamide (INCI : Acrylates/Octylacrylamide Copolymer) et au plus 2,5 % en poids de glycérol, par rapport au poids total de la préparation.

17. Utilisation ou préparation selon l'une des revendications précédentes, **caractérisée en ce que** la préparation est anhydre.

18. Utilisation ou préparation selon l'une des revendications précédentes, **caractérisée en ce que** la préparation est exempte de dioxyde de titane, d'oxyde de zinc et de polysilicone-15.

19. Utilisation ou préparation selon l'une des revendications précédentes, **caractérisée en ce que** la préparation est stockée avec un récipient de gaz comprimé en aérosol et est pulvérisée à l'aide d'un gaz propulseur.

20. Utilisation ou préparation selon la revendication 20, **caractérisée en ce que** l'on utilise comme gaz propulseur du propane, du n-butane, de l'isobutane ou leurs mélanges.
